# EUROPÄISCHE PATENTSCHRIFT

(11) **EP 0 868 980 B1**
(45) Veröffentlichungstag und Bekanntmachung des Hinweises auf die Patenterteilung: **03.09.2003**
(21) Anmeldenummer: 98810286.9
(22) Anmeldetag: 03.04.1998
(51) Int. Cl.: B25C 3/00, B25G 1/08, B25C 11/00

(54) **Vorrichtungen zum Handhaben von Gegenständen mit einem Kopf und einem Schaft, wie Schrauben, Nägel und dergleichen**
Handling device for articles such as screws or nails and the like
Dispositif de manipulation pour des articles tels que vis, clous et similaire

(30) Priorität: 05.04.1997 DE 19714055
(43) Veröffentlichungstag der Anmeldung: 07.10.1998
(73) Patentinhaber: Medartis AG, 4051 Basel (CH)
(72) Erfinder: Roth, Michael, 79285 Ebringen (DE); Rath-Prazak, Paul, 79206 Breisach (DE)
(74) Vertreter: Heinen, Detlef

(56) Entgegenhaltungen:
- CH-A- 270 065
- DE-C- 38 035
- US-A- 750 605
- US-A- 3 009 155

## Beschreibung

### Anwendungsgebiet der Erfindung

Die Erfindung betrifft Vorrichtungen zum Handhaben von Gegenständen mit einem Kopf und einem Schaft, wie Schrauben, Nägel und dergleichen, mit einem Handstück, das eine Kammer zur Aufnahme der Gegenstände aufweist, wobei sich von einer Innenseite der Kammer zu einer Ausgabeseite des Handstücks eine Führungsausnehmung erstreckt, die einen an die Aussenkontur der Gegenstände angepassten Querschnitt aufweist und am ausgabeseitigen Ende über einen gegenüber einem an die Kammer angrenzenden Abführabschnitt abgewinkelten Endabschnitt verfügt.

### Stand der Technik

Eine derartige Vorrichtung ist aus der US-A-3,101,477 bekannt. Bei dieser Vorrichtung zum Handhaben von Reissnägeln mit einem Kopf und einem Schaft ist ein Handstück vorgesehen, das eine Kammer zur Aufnahme der Reissnägel aufweist. Von der Innenseite der Kammer zu einer Ausgabeseite des Handstücks erstreckt sich eine Führungsausnehmung, die einen an die Aussenkontur der Reissnägel angepassten Querschnitt aufweist und am ausgabeseitigen Ende über einen gegenüber einem an die Kammer angrenzenden Abführabschnitt abgewinkelten Endabschnitt verfügt. Der Abführabschnitt geht an einem Ende mit einer Abrundung in die Kammer über, während sich an seinem anderen Ende ein abgerundeter Übergangsbereich zu dem gerade verlaufenden Endabschnitt anschließt. Weiterhin verfügt die gattungsgemässe Vorrichtung über einen knopfartig ausgebildeten Schieber, der entlang der Führungsausnehmung von dem Abführabschnitt durch den abgerundeten Übergangsabschnitt in Richtung des Endabschnitts verschiebbar ist. Der Schieber dient zum Überführen von Reissnägeln aus der Kammer in den Endabschnitt. Die gattungsgemässe Vorrichtung erfordert jedoch zur Ausgabe von einzelnen Reissnägeln den Schieber sowie eine verhältnismässig aufwendig herzustellende Anordnung von Zuführschlitzen im Mündungsbereich des Abführabschnittes in die Kammer.

Aus der US-A-2,431,831 ist eine Vorrichtung zum Handhaben von Nägeln mit einem Kopf und einem Schaft bekannt, die eine an einem Trageteil befestigte Vorratskammer aufweist, in die die Nägel einfüllbar sind. An die Vorratskammer schliesst sich eine von zwei Seitenteilen begrenzte Führungsausnehmung an, die einen fluchtend mit dem Boden der Vorratskammer ausgerichteten Abführabschnitt und einen über einen S-förmig gebogenen Übergangsabschnitt mit dem Abführabschnitt in Verbindung stehenden Endabschnitt aufweist. Dabei sind die Vorratskammer, der Abführabschnitt und der Endabschnitt gegenüber der Vertikalen etwa gleich geneigt angeordnet und der S-förmige Übergangsabschnitt so ausgerichtet, dass sein Mittelteil gegenüber dem Abführabschnitt und dem Endabschnitt verstärkt in Richtung der Vertikalen geneigt ist. An einem ausgabeseitigen Ende des Endabschnitts ist ein Mechanismus zur Entnahme der Nägel vorgesehen. Diese Vorrichtung ist jedoch nicht zur Ausgabe von einzelnen Gegenständen direkt aus dem ausgabeseitigen Ende des Endabschnitts eingerichtet.

Aus der US-A-2,775,766 ist eine Vorrichtung zum Handhaben von Nägeln mit einem Kopf und einem Schaft bekannt, die über eine Anzahl von parallel zueinander verlaufenden und in der Dicke der Nägelschäfte voneinander beabstandeten Rohrteilen verfügt. An einem ausgabeseitigen Ende der Vorrichtung weisen die Rohrteile eine Abschrägung auf. Zwischen den Rohrteilen sind Nägel einführbar, wobei sich die Schäfte durch die Engführung zwischen den Rohrteilen erstrecken und die Köpfe in einem aufgeweiteten Bereich zwischen den Rohrteilen angeordnet sind. Durch Verkippen der Vorrichtung gegenüber der Horizontalen gleiten die Nägel zwischen den Rohrteilen in Richtung deren ausgabeseitigen Enden, wobei sie durch die dort vorgesehene Abschrägung von einem Querstück im Bereich der Schäfte zurückgehalten verkippen und von Hand entnehmbar sind.

Aus der DE-U-93 03 687 ist eine Handhabungshilfe für Schnellbauschrauben bekannt, welche ein mit den Fingern aufschiebbares Halteteil besitzt. Das Halteteil weist zwei parallel zueinander verlaufende Magnetstreifen auf, an denen metallische Schnellbauschrauben anhaften können. Zwischen den Magnetstreifen erstreckt sich eine T-förmige Nut, in die ein Magazinstreifen einschiebbar ist, an dem über Laschen die Schnellbauschrauben befestigt sind. Das Halteteil verfügt am ausgabeseitigen Ende über eine Mulde, in die ein Schaft eines zu haltenden Gegenstands einfügbar ist.

Aus der DE-A-39 30 999 ist eine Zuführeinrichtung für ein vorzugsweise an einem Roboter zu befestigendes Gerät zum Eintreiben eines Befestigungsmittels bekannt, bei der über einen Kettenmechanismus in eine Führungsausnehmung eingebrachte Befestigungsmittel um eine Umlenkrolle des Kettenmechanismus herum zu einer Ausgabeseite überführbar sind. An der Ausgabeseite ist beispielsweise ein Schraubwerkzeug vorgesehen.

Aus der DE-U-94 12 333 ist ein Nagel- und Schraubenhalter in Gestalt einer Zange bekannt, an deren Greifbacken eingefräste Kerben zur Aufnahme von Nägeln oder Schrauben eingebracht sind. Zum sicheren Halten der zu handhabenden Gegenstände ist eine Druckfeder vorgesehen, welche die Greifbacken zusammendrückt.

Weitere herkömmliche Vorrichtungen zum Handhaben von insbesondere verhältnismässig kleinen, einen Kopf und einen Schaft aufweisenden Gegenständen, wie Schrauben, Nägel und dergleichen, sind z.B. als Pinzetten ausgeführt, mit denen ein Gegenstand mit den Pinzettenspitzen im Bereich des Schafts oder des Kopfes greifbar und z.B. in eine von mehreren gitterartig angeordneten Schraubenaufnahme-Ausnehmungen eines Schrauben-Aufnahmeteils, wie es auf dem Gebiet der Chirurgie zum Vorhalten von Knochenschrauben während der Operation verwendet wird, einsetzbar ist. Grundsätzlich sind zwar mit diesen Vorrichtungen Gegenstände mit einem Kopf und einem Schaft handhabbar. Allerdings ist z.B. das Einsetzen insbesondere kleiner Gegenstände in die Schraubenaufnahme-Ausnehmungen eines Schrauben-Aufnahmeteils sehr zeitraubend und erfordert eine konzentrierte, manuell geschickte Arbeitsweise. Dies ist eventuell nach vielstündiger Tätigkeit im Operationssaal nur mehr eingeschränkt möglich.

### Aufgabe der Erfindung

Der Erfindung liegt die Aufgabe zugrunde, Vorrichtungen der eingangs genannten Art zu schaffen, mit denen Gegenstände, die einen Kopf und einen Schaft aufweisen, mit einfachen Handgriffen - auch unter erschwerten Arbeitsumständen - zuverlässig handhabbar sind.

### Übersicht über die Erfindung

Diese Aufgabe wird bei einer Vorrichtung eines ersten Typs erfindungsgemäss dadurch gelöst, dass der Endabschnitt wenigstens in seinem an den Abführabschnitt angrenzenden Bereich spitzwinklig zum Abführabschnitt ausgerichtet ist sowie entgegen der Zuführrichtung eines Gegenstands von der Kammer in den an den Endabschnitt angrenzenden vorderseitigen Bereich des Abführabschnitts, und damit in Richtung des Abführabschnitts, geneigt verläuft.

Dadurch, dass bei der Vorrichtung des ersten Typs der Endabschnitt spitzwinklig zum Abführabschnitt ausgerichtet ist sowie in Richtung des Abführabschnitts geneigt verläuft, ist eine Ausgabesperre geschaffen, die ein Herausgleiten der Gegenstände, z.B. bei einer unkontrollierten Verkippung des Handstücks gegenüber der Horizontalen, verhindert. Die Ausgabe von Gegenständen erfolgt erst nach einer kontrollierten Handhabung, indem die Gegenstände um die Abwinklung herum geführt werden.

Die Aufgabe wird mit einer Vorrichtung eines zweiten Typs erfindungsgemäss dadurch gelöst, dass der Endabschnitt wenigstens in seinem an den Abführabschnitt angrenzenden Bereich rechtwinklig zum Abführabschnitt ausgerichtet ist und dass im Übergangsbereich zwischen dem Abführabschnitt und dem Endabschnitt als Kopf-Einrastmittel ein sich in Verlängerung des Endabschnitts erstreckender Sackfortsatz und/oder eine dem Abführabschnitt gegenüber liegende Ausbuchtung vorgesehen sind.

Durch die rechtwinklige Ausrichtung des Endabschnitts zum Abführabschnitt und das Vorsehen eines Sackfortsatzes und/oder einer Ausbuchtung kann ein Kopf eines Gegenstands einrastartig im Übergangsbereich vom Abführabschnitt in den Endabschnitt eingleiten, so dass sowohl das unbeabsichtigte Nachrutschen von nachfolgenden Gegenständen blockiert als auch ein unbeabsichtigtes Verlieren der Gegenstände verhindert ist. Erst nach kontrollierter Ausgabebewegung wird der vorderseitige Gegenstand aus dem Endabschnitt ausgeführt, wobei der nachfolgende Gegenstand nunmehr in den Sackfortsatz und/oder die Ausbuchtung eingleitet.

Durch das Vorsehen eines Handstücks, das über eine Kammer zur Aufnahme der Gegenstände verfügt, wobei die Gegenstände über die an die Gegenstände konturangepasste Führungs-Ausnehmung mit einem abgewinkelten Endabschnitt zu der Ausgabeseite überführbar sind, ist die zuverlässige Handhabung mit einfachen Handgriffen dadurch erzielt, dass zum einen eine gewisse Anzahl von Gegenständen in die Kammer einfüllbar ist und die Gegenstände, z.B. durch Verkippen gegenüber der Horizontalen, in den Abführabschnitt der Führungs-Ausnehmung in einer Aufreihung bis zu dem abgewinkelten Endabschnitt überführbar sind. Die aufgereihten Gegenstände sind z.B. durch Verkippen des Handstücks um eine durch die Abwinklung gebildete Ausgabesperre einzeln nacheinander herumführbar, so dass eine mit einfachen Handgriffen kontrollierbare Ausgabe, beispielsweise zum Einfügen der Gegenstände in Schrauben-Aufnahmeausnehmungen eines Schrauben-Aufnahmeteils, geschaffen ist.

Bei einer zweckmässigen Weiterbildung ist die Ausgabeseite des Handstücks spitzenartig ausgebildet, so dass die Gegenstände auch unter räumlich relativ beengten Verhältnissen, z.B. in eng benachbarte Schrauben-Aufnahmeausnehmungen, einfügbar sind. Um eine effektiv wirkende Ausgangssperre zu schaffen, ist es zweckmäßig, dass der Abführabschnitt und der Endabschnitt in einem Stossbereich spitzwinklig aufeinander ausgerichtet sind. Die kontrollierte Ausgabe aus dem Endabschnitt wird durch einen in Richtung des Abführabschnitts gekrümmt ausgebildeten Endabschnitt erleichtert, indem das Handstück in einer abrollartigen Bewegung gehandhabt wird.

Es ist zweckmässig, im spitzwinkligen Übergangsbereich zwischen Abführabschnitt und Endabschnitt einen sich in Verlängerung des Endabschnitts erstreckenden Sackfortsatz und/oder eine Ausbuchtung des Endabschnitts vorzusehen, in die ein Kopf eines Gegenstands einrastartig eingleiten kann, so dass sowohl das unbeabsichtigte Nachrutschen von nachfolgenden Gegenständen blockiert als auch ein unbeabsichtigtes Verlieren der Gegenstände verhindert ist. Erst nach einer kontrollierten Ausgabebewegung wird der vorderseitige Gegenstand aus dem Endabschnitt ausgeführt, wobei der nachfolgende Gegenstand nunmehr in den Sackfortsatz und/oder die Ausbuchtung eingleitet.

Das Überführen der Gegenstände vom Abführabschnitt in den Endabschnitt wird durch das Vorsehen eines gekrümmten Übergangsabschnitts erleichtert, ohne dass die Wirkung der Abwinklung als Ausgabesperre nachteilig beeinflusst wird.

Um Gegenstände mit Schäften unterschiedlicher Länge und/oder Durchmesser, jedoch ansonsten gleicher Aussenkontur im Bereich der Köpfe mit einem Handstück handhaben zu können, ist es zweckmässig, dass der die Schäfte aufnehmende Teil der Führungsausnehmung zu der der Kammer gegenüber liegenden Seite des Handstücks geöffnet ist.

Damit die Gegenstände zuverlässig in die Führungsausnehmung einfügbar sind, ist es zweckmässig, dass sich die Führungsausnehmung an den Grund der Kammer anschliesst, wobei der Kammergrund spitzwinklig ausgebildet ist. Damit es im Mündungsbereich des Abführabschnitts der Führungsausnehmung der Kammer zu keinen Verklemmungen kommt, ist es zweckmässig, die Führungsausnehmung im Mündungsbereich in die Kammer aufgeweitet auszugestalten.

Falls in die Kammer eine Anzahl von Gegenständen eingebracht werden soll, die das Fassungsvermögen des Abführabschnitts zwischen Kammer und Endabschnitt übersteigt, ist es zweckmässig, einen Deckel vorzusehen, mit dem die Kammer verschliessbar ist. Weitere zweckmässige Ausgestaltungen und Vorteile der Erfindung sind in den abhängigen Ansprüchen definiert.

Die Fertigung des Handstücks ist dadurch wesentlich vereinfacht, dass das Handstück aus zwei aneinander fügbaren Handstückteilen aufgebaut ist. Ein essentieller Vorteil der erfindungsgemässen Vorrichungen besteht insbesondere darin, dass die zu handhabenden Gegenstände - z.B. Implantatschrauben - beim Nachfüllen direkt aus der Verpackung in die Einfüllkammer gegeben werden. Jegliche Fremdberührung, z.B. mit den Händen oder einer Pinzette, erübrigt sich. Damit wird die Gefahr der Kontamination oder die Oberflächenbeschädigung der zu handhabenden Gegenstände ausgeschlossen. In den erfindungsgemässen Vorrichungen sind keinerlei bewegliche Vorrichtungsteile vorhanden, welche Ursache für eine Beschädigung der Oberfläche der zu handhabenden Gegenstände sein könnten.

### Kurzbeschreibung der Zeichnungen

Es zeigen:

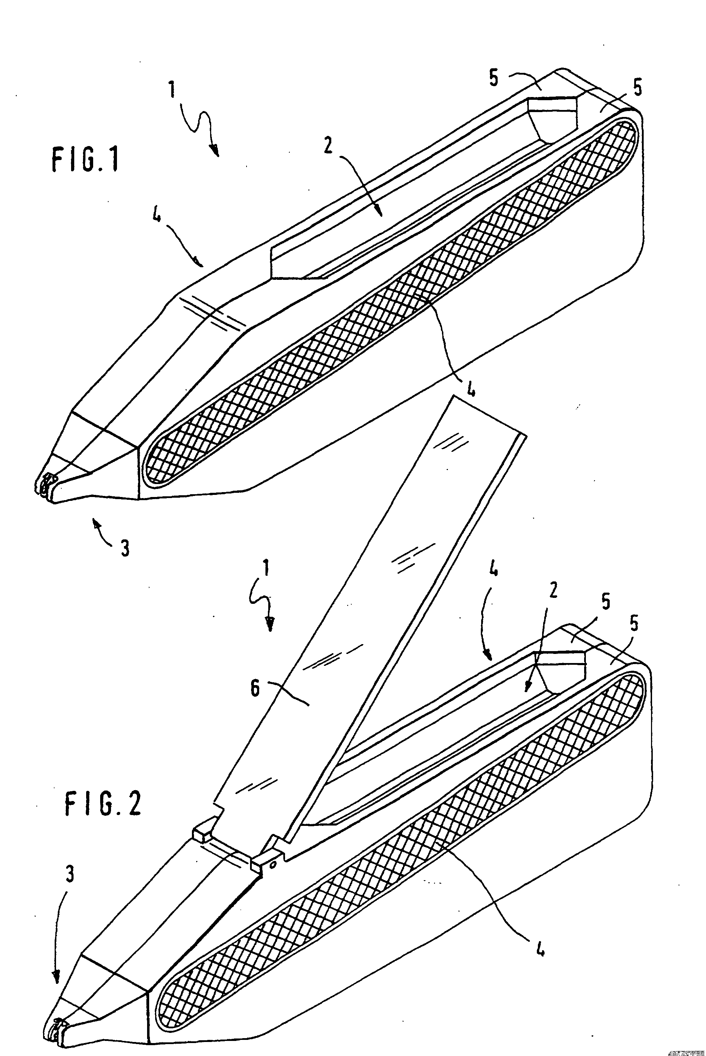

- Figur 1:: als Perspektivansicht eine erfindungsgemässe Vorrichtung mit einem aus zwei Handstückteilen zusammengesetzten Handstück, das eine offenbleibende Einfüllkammer und eine an einer Ausgabeseite ausgebildete Handstückspitze aufweist,
- Figur 2:: als Perspektivansicht die Vorrichtung gemäss Figur 1 mit einer verschliessbaren Einfüllkammer,
- Figur 3:: als Seitenansicht ein Handstückteil der Vorrichtung gemäss Figur 1 mit einer sich zwischen Einfüllkammer und der Handstückspitze erstreckenden Führungsausnehmung,

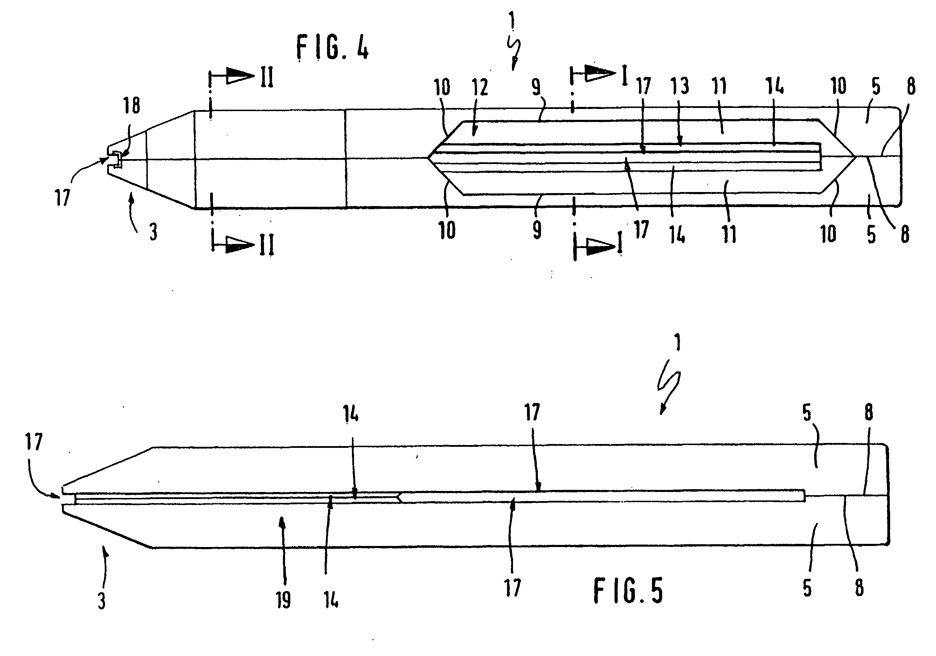

- Figur 4:: als Draufsicht die Einfüllkammer der Vorrichtung gemäss Figur 1,
- Figur 5:: als Draufsicht das Handstück gemäss Figur 1 auf die der Einfüllkammer gegenüber liegende Seite,

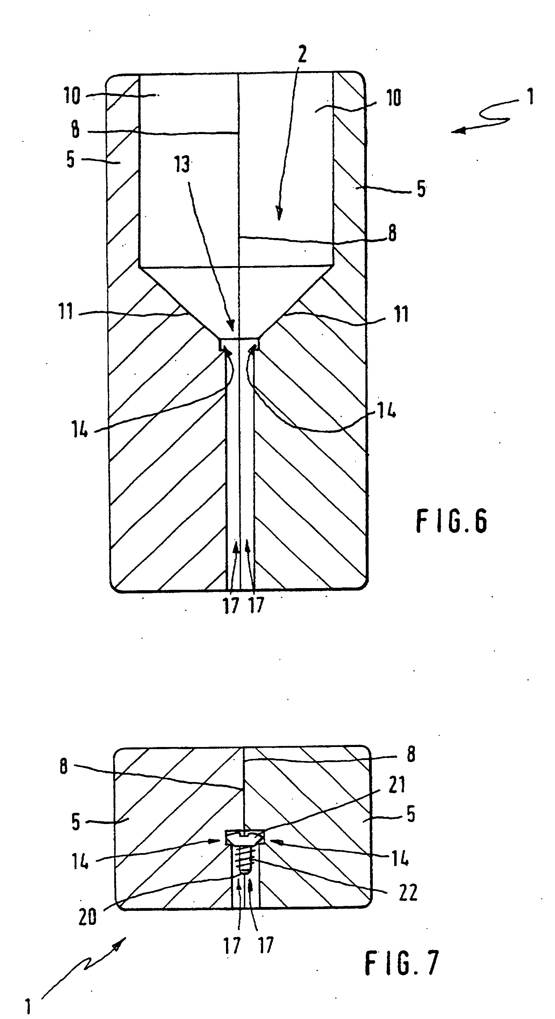

- Figur 6:: einen Schnitt entlang der Linie I-I gemäss Figur 4,
- Figur 7:: einen Schnitt entlang der Linie II-II gemäß Fig. 4,
- Figur 8:: als vergrösserte Seitenansicht das Handstückteil gemäss Figur 3 im Bereich der Handstückspitze,
- Figur 9:: als vergrösserte Perspektivdarstellung die Handstückspitze der Vorrichtung gemäss Figur 1,

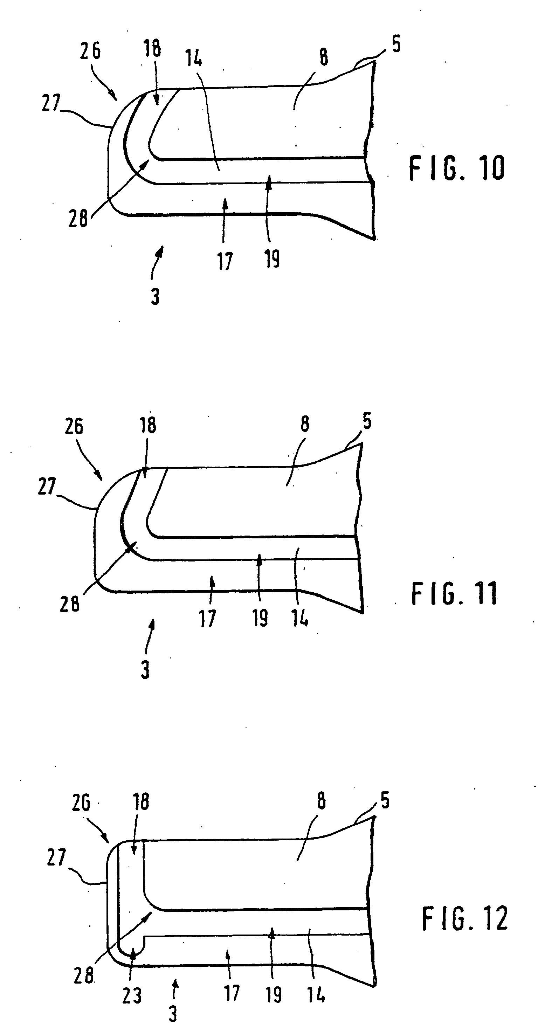

- Figur 10:: als Seitenansicht eine weitere Abwandlung der Handstückspitze,
- Figur 11:: als Seitenansicht die Handstückspitze mit einem gerade ausgebildeten Endabschnitt,
- Figur 12:: als Seitenansicht die Handstückspitze mit zueinander rechtwinklig ausgerichtetem Endabschnitt und Abführabschnitt,

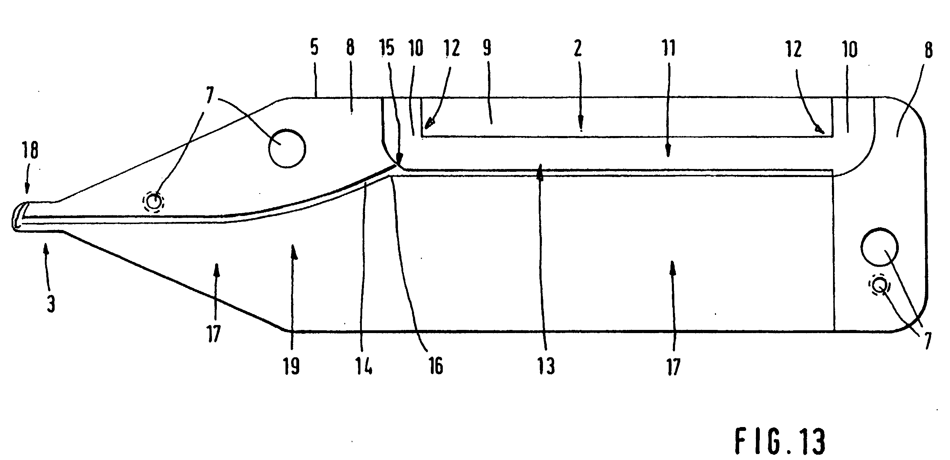

- Figur 13:: als Seitenansicht ein Handstückteil mit einer gegenüber der Vorrichtung gemäss Figur 1 weniger tief eingebrachten Einfüllkammer,

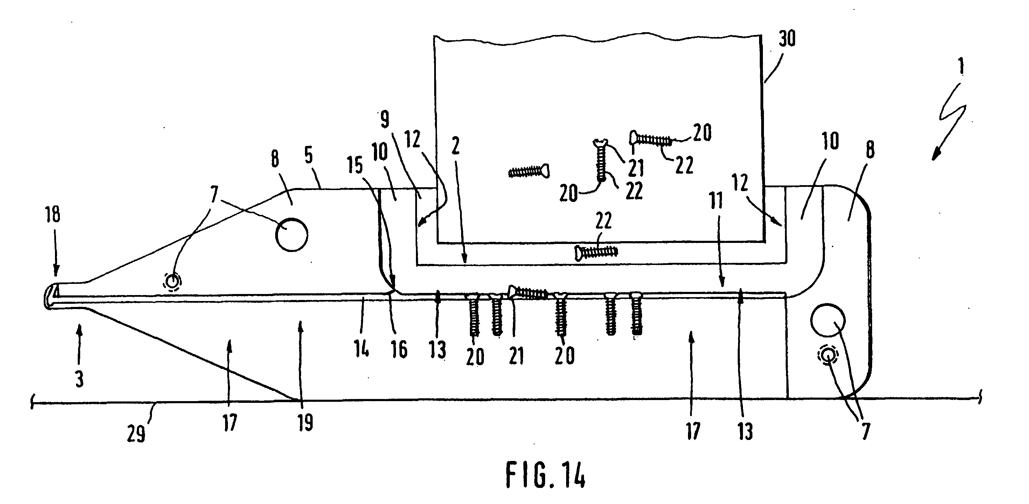

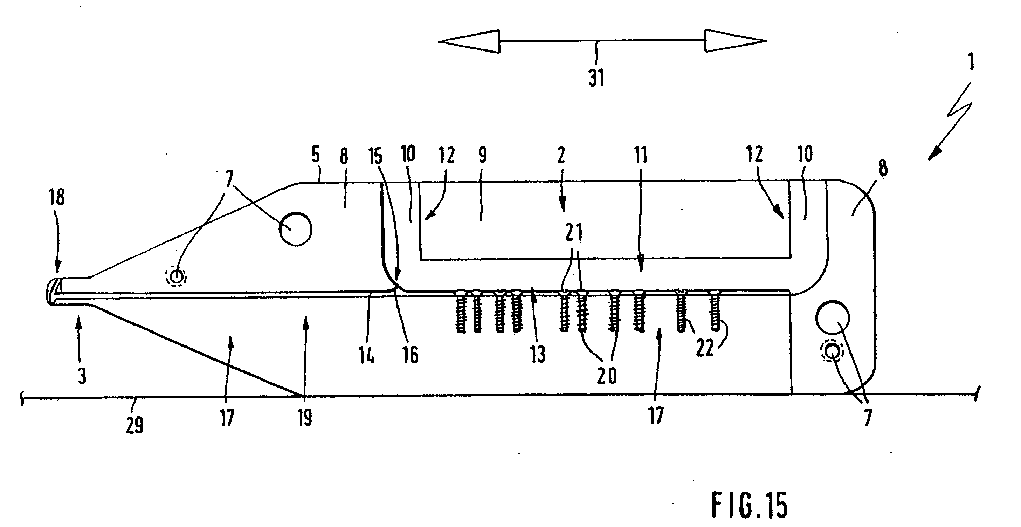

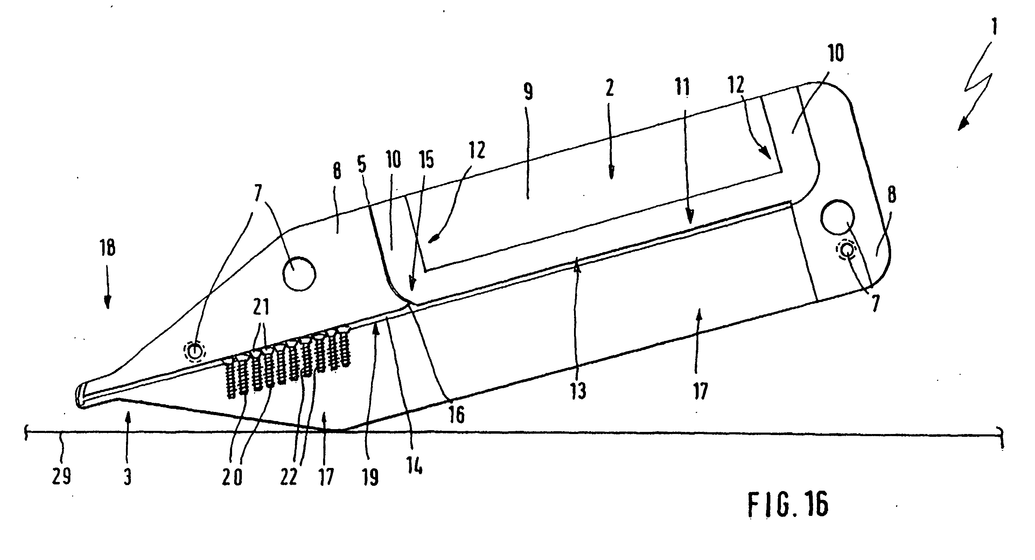

- Figuren 14 bis 18:: als Seitenansichten auf ein Handstückteil zum Erläutern der Handhabung des Handstücks gemäss Figur 1 beim Einsetzen von Schrauben in ein Schrauben-Aufnahmeteil sowie

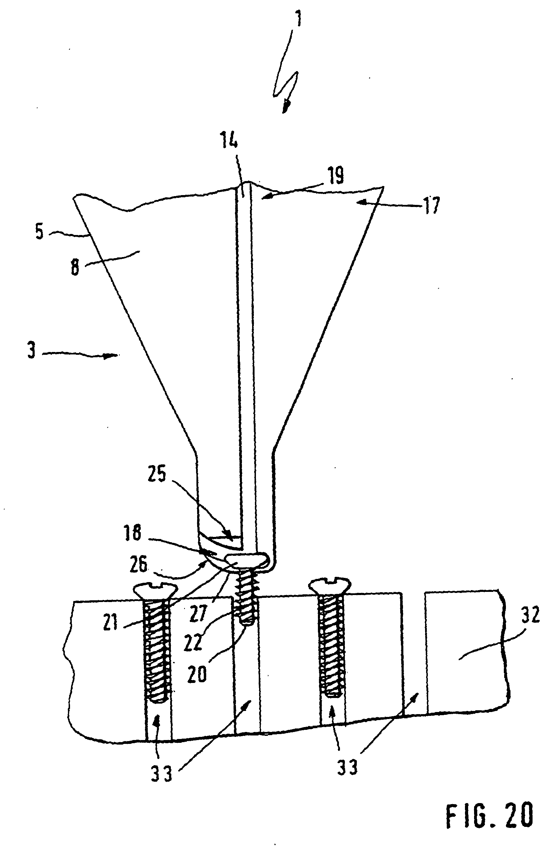

- Figuren 19 und 20:: als Seitenansicht auf ein Handstückteil zur Erläuterung der Handhabung des Handstücks gemäss Figur 1 beim Aufnehmen von Schrauben aus einem Schrauben-Aufnahmeteil.

### Detaillierte Beschreibung von Ausführungsbeispielen

Mit Bezug auf die Figuren werden nachstehend bevorzugte Ausführungsbeispiele erfindungsgemässer Vorrichtungen beschrieben.

### Figur 1

Das Handstück **1** ist im Bereich einer Einfüllkammer **2** als Kammer länglich quaderförmig ausgestaltet und im Bereich einer Handstückspitze **3** als Ausgabeseite sich verjüngend ausgebildet. Die Einfüllkammer **2** ist hier offenbleibend ausgestaltet. An Seitenflächen des Handstücks **1** sind Griffleisten **4** angebracht, die über eine Erhebungen und Vertiefungen aufweisende Oberfläche verfügen und zweckmässigerweise farbig ausgestaltet sind, um einem Benutzer den Anwendungsbereich des Handstücks **1** - zum Handhaben von z.B. Schrauben einer bestimmten Grösse - zu indizieren. Das Handstück **1** ist aus zwei bündig aneinander fügbaren Handstückteilen **5** aufgebaut.

### Figur 2

An dem weitergebildeten Handstück **1** ist ein vorzugsweise durchsichtiger Klappdeckel **6** zum Verschliessen der Einfüllkammer **2** angebracht. Die Ausführungsform dieses Handstücks **1** ist insbesondere in Anwendungsfällen zweckmässig, bei denen während der Handhabung eine Anzahl von in die Einfüllkammer **2** eingebrachten Gegenständen in dieser verbleibt, da der Klappdeckel **6** in geschlossener Stellung ein Herausfallen der in der Einfüllkammer **2** verbliebenen Gegenstände unterbindet.

### Figur 3

Das hier gezeigte Handstückteil **5** besitzt Einrichtungen **7** zum ausgerichteten Verbinden beider Handstückteile **5**. Die Einrichtungen **7** sind - z.B. durch in Ausrichtausnehmungen eingreifende Vorsprünge zum passgenauen Ausrichten und durch Befestigungsausnehmungen mit gegenüber liegenden Innengewindeabschnitten - zum lösbaren Verschrauben gebildet.

Zum Ausbilden der Einfüllkammer **2** sind in die Handstückteile **5** in ihren aufeinander zuweisenden Innenseitenflächen **8** Einfüllkammer-Ausnehmungen eingebracht, die - z.B. von einem parallel zu der Innenseitenfläche **8** ausgerichteten Parallel-Wandabschnitt **9** und einem sich zwischen dem Parallel-Wandabschnitt **9** und der Innen-Seitenfläche **8** erstreckenden Schräg-Wandabschnitt **10 -** begrenzt sind, so dass die Einfüllkammer **2** am Boden-Innenrand **11** und den Seiten-Innenrändern **12** sich verjüngend ausgebildet ist.

Der Verjüngungswinkel ist so ausgebildet, dass in die Einfüllkammer **2** eingebrachte Gegenstände mit Kopf und Schaft bei einer im wesentlichen horizontalen Ausrichtung des Boden-Innenrands **11** durch Einwirkung der Schwerkraft selbsttätig im Einfüllkammer-Grund **13** zu liegen kommen. Beispielsweise liegt der Verjüngungswinkel zwischen etwa 60° und etwa 120°, vorzugsweise zwischen etwa 80° und 100°.

Weiterhin ist in jedes Handstückteil **5** eine sich von der Einfüllkammer **2** zur Handstückspitze **3** erstreckende Kopf-Führungsnut **14** einer Führungsausnehmung eingebracht, die sich auch entlang des Einfüllkammer-Grunds **13** der Einfüllkammer **2** erstreckt und so angeordnet ist, dass der Boden-Innenrand **11** der Einfüllkammer **2** im Bereich des Einfüllkammer-Grunds **13** zur Kopf-Führungsnut **14** geöffnet ist. Es ist zweckmässig, dass die Kopf-Führungsnut **14** im Mündungsbereich **15** in die Einfüllkammer **2** im Querschnitt mittels einer Einführ-Abrundung **16** zum Erleichtern des Eintritts eines Kopfs eines Gegenstands aufgeweitet ist.

Jedes Handstückteil **5** weist an der der Einfüllkammer **2** gegenüberliegenden Seite der Kopf-Führungsnut **14** eine sich bis zu der der Einfüllkammer **2** gegenüberliegenden Aussenseite der Handstückteile **5** erstreckende Schaftführungs-Ausnehmung **17** einer Führungs-Ausnehmung auf, die an die Kopf-Führungsnut **14** angrenzt. Die Schaftführungs-Ausnehmung **17** erstreckt sich entlang der Kopf-Führungsnut **14** bis in die Handstückspitze **3** und tritt gegen die Innenseitenfläche **8** um einen gegenüber der Tiefe der Kopf-Führungsnut **14** geringeren Versatz zurück.

Im Bereich der Handstückspitze **3** weist die Kopf-Führungsnut **14** einen abgewinkelten Endabschnitt **18** auf, der hier spitzwinklig zu einem sich zwischen der Einfüllkammer **2** und dem Endabschnitt **18** erstreckenden Abführabschnitt **19** ausgerichtet ist, wobei der Endabschnitt **18** und der Abführabschnitt **19** in ihrem Stossbereich so ausgebildet sind, dass eine Ausgabesperre gebildet ist, die den selbsttätigen Austritt von Gegenständen aus der durch die Kopf-Führungsnuten **14** und die Schaft-Führungsausnehmungen **17** gebildeten Führungsausnehmung verhindert.

### Figur 4

Ersichtlich ist das aus den zusammengefügten Handstückteilen **5** bestehende Handstück **1** mit der Einfüllkammer **2**. Im Einfüllkammer-Grund **13** ist für die Köpfe der handzuhabenden Gegenstände durch die im Übergang von den Kopf-Führungsnuten **14** zu den Schaftführungs-Ausnehmungen **17** ausgebildeten Schultern eine Auflagefläche geschaffen, während die Schaftführungs-Ausnehmungen **17** den Eintritt von Schäften der Gegenstände zulassen.

### Figur 5

Es ist ersichtlich, dass sich die Schaftführungs-Ausnehmungen **17** über die gesamte Länge der Kopf-Führungsnut **14** von der Einfüllkammer **2** bis in die Handstückspitze **3** erstrecken.

### Figur 6

Ersichtlich ist, wie die Kopf-Führungsnuten **14** und die Schaftführungs-Ausnehmungen **17** der Handstückteile **5** sich zur Führungsausnehmung ergänzen, deren Querschnitt an die Aussenkontur der zu handhabenden Gegenstände angepasst ist.

### Figur 7

Bei diesem Ausführungsbeispiel befindet sich eine Schraube **20** im Handstück **1**. Der Schraubenkopf **21** liegt an den zwischen den Kopf-Führungsnuten **14** und den Schaftführungs-Ausnehmungen **17** ausgebildeten Schultern an, während der Schraubenschaft **22** in die Schaftführungs-Ausnehmungen **17** eingetreten ist. Die Schraube **20** wird mit geringem Spiel durch eine Führungsausnehmung mit einem Querschnitt geführt, welcher der Aussenkontur der Schraube **20** angepasst ist. Diese Führungsausnehmung bildet sich aus den Kopf-Führungsnuten **14** und den Schaftführungs-Ausnehmungen **17.**

### Figur 8

Im Übergangsbereich zwischen dem Abführabschnitt **19** und dem entgegen der Zuführrichtung eines Gegenstands von der Einfüllkammer **2** in den an den Endabschnitt **18** angrenzenden vorderseitigen Bereich des Abführabschnitts **19** - also in Richtung des Abführabschnitts **19** gekrümmt verlaufenden Endabschnitt **18** der Kopf-Führungsnut **14** - ist in Verlängerung des Endabschnittes **18** ein Sackfortsatz **23** ausgebildet, der im wesentlichen rechtwinklig zu dem Abführabschnitt **19** ausgerichtet ist. Der Endabschnitt **18** ist mit einer Ausbuchtung **24** in einem dem Abführabschnitt **19** gegenüberliegenden Bereich vergrössert. Weiterhin ist zwischen dem Endabschnitt **18** und dem Abführabschnitt **19** der Kopf-Führungsnut **14** in die Innenseitenfläche **8** eine Übergangsausnehmung **25** eingebracht, deren Tiefe vorzugsweise der Tiefe der Schaftführungs-Ausnehmung **17** entspricht.

### Figur 9

Eine Auflageseite **26** der Handstückspitze **3** ist entsprechend dem Verlauf des Endabschnitts **18** der Kopf-Führungsnut 14 ausgestaltet, so dass zwischen dem Endabschnitt **18** und einer vorderen Aussenseite **27** des Handstücks **1** im Bereich der Auflageseite **26** eine im wesentlichen gleichbleibende Rest-Materialstärke vorliegt.

### Figur 10

Die zwischen dem Endabschnitt **18** und dem Abführabschnitt **19** gelegene Kopf-Führungsnut **14** weist einen im wesentlichen gleich bleibenden Querschnitt und Innenseitenflächen **8** auf, die auch im Zwischenbereich zwischen dem Endabschnitt **18** und dem Abführabschnitt **19** flächig aufeinanderliegen. Ein Übergangsbereich **28** zwischen dem Endabschnitt **18** und dem Abführabschnitt **19** der Kopf-Führungsnut **14** ist rundlich ausgebildet, wobei auch bei dieser Abwandlung der Endabschnitt **18** gekrümmt ist.

### Figur 11

Hier ist der Endabschnitt **18** gerade ausgebildet.

### Figur 12

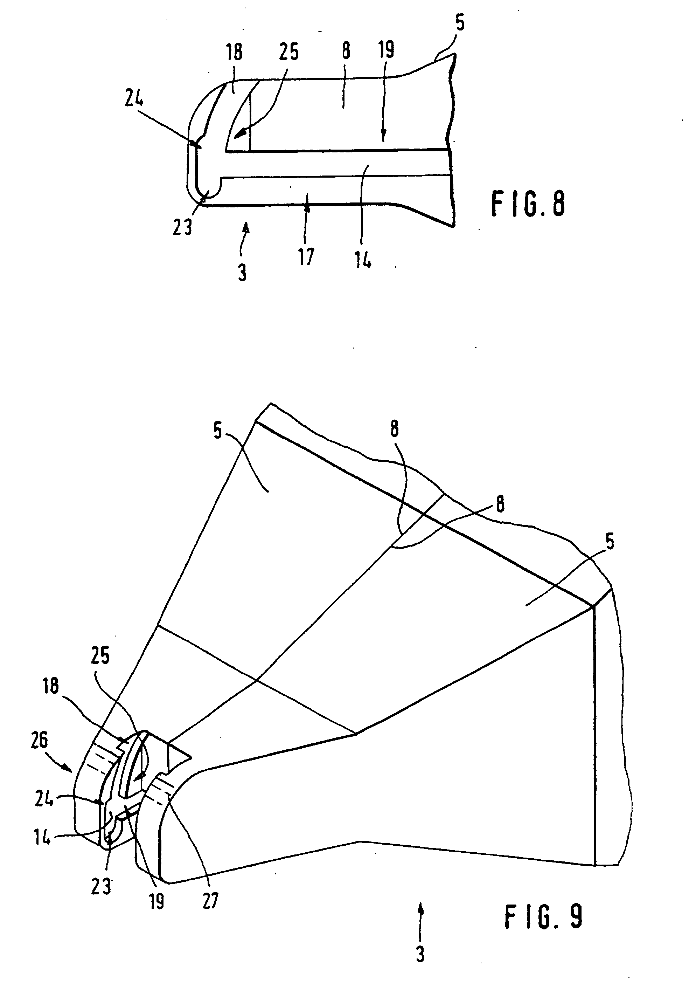

Bei dieser Abwandlung sind der Endabschnitt **18** und der Abführabschnitt **19** rechtwinklig zueinander ausgerichtet, wobei der Endabschnitt **18** gerade ausgebildet ist. Entsprechend der Ausgestaltung gemäss Figur 8 ist dem Endabschnitt **18** gegenüberliegend ein an den Abführabschnitt **19** angrenzender Sackfortsatz **23** ausgebildet. Die Innenseitenflächen **8** der Handstückteile **5** liegen bis an die Kopf-Führungsnut **14** grenzend plan aneinander an, wobei die dem Sackfortsatz **23** gegenüberliegende abgewendete Wandung der Kopf-Führungsnut **14** einen halbseitig gekrümmten Übergangsbereich **28** bildet.

### Figur 13

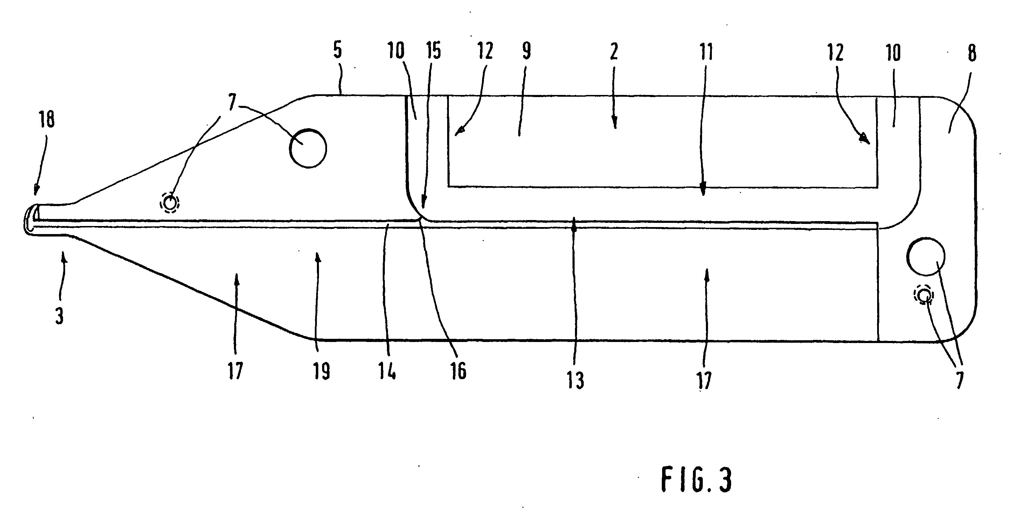

Bei der hier gezeigten Abwandlung der Handstückteile **5** ist die Tiefe der Einfüllkammer **2** zum Einfüllkammer-Grund **13** kleiner als bei dem Ausführungsbeispiel gemäss Figur 3. Die Kopf-Führungsnut **14** zwischen dem Mündungsbereich **15** und der Handstückspitze **3** ist in einem sich an die Einfüllkammer **2** anschliessenden Bereich des Abführabschnitts **19** gekrümmt, um ein Verklemmen von eingeführten Gegenständen zu vermeiden.

### Figur 14

Das Handstück **1** mit seinen beiden Handstückteilen **5** ist mit seiner der Einfüllkammer **2** gegenüberliegenden Aussenseite auf eine im wesentlichen horizontal ausgerichtete Auflagefläche **29** aufgelegt. In die Einfüllkammer **2** ragt eine geöffnete Seite eines mit Schrauben **20** gefüllten Bevorratungs-Behälters **30**, wie ein Kunststoffbeutel oder Kunststoffschachtel hinein. Einige der Schrauben **20** sind bereits durch die Schräg-Wandabschnitte **10** des Boden-Innenrands **11** im Einfüllkammer-Grund **13** der Einfüllkammer **2** angeordnet und liegen mit ihren Schraubenköpfen **21** in den Kopf-Führungsnuten **14**. Die Schraubenschäfte **22** einiger dieser Schrauben **20** sind bereits in die Schaftführungs-Ausnehmungen **17** eingetreten.

### Figur 15

Nun sind die in die Einfüllkammer **2** eingefüllten Schrauben **20**, z.B. wie durch Pfeil **31** angedeutet, durch eine Hin- und Herbewegung längs der Auflagefläche **29** in eine Lage gebracht. In dieser Lage sind alle Schraubenschäfte **22** in die Schaftführungs-Ausnehmungen **17** eingetreten.

### Fiaur 16

Das Handstück **1** befindet sich jetzt in einer gegenüber der Auflagefläche **29** gekippten Stellung. Die Schrauben **20** sind, der Hangabtriebskraft folgend, in den Abführabschnitt **19** der Kopf-Führungsnuten **14** eingetreten.

### Figur 17

Man sieht den Spitzenbereich **3** des Handstücks **1** mit dem an den Endabschnitt **18** angrenzenden Bereich des Abführabschnitts **19**, in welchem die Schrauben **20** aufeinander folgend angeordnet sind. Das Handstück **1** ist hier im wesentlichen rechtwinklig zu einem im wesentlichen horizontal liegenden Schrauben-Aufnahmeteil **32** angeordnet, das über eine Anzahl von regelmässig beabstandeten Schrauben-Aufnahmeausnehmungen **33** verfügt, in welche die Schrauben **20** mit ihren Schraubenschäften **22** einzubringen sind. Die in Richtung des Endabschnitts **18** vorderste Schraube **20** ist mit ihrem Schraubenkopf **21** vom Abführabschnitt **19** in den Sackfortsatz **23** und in die Ausbuchtung **24** des Endabschnitts **18** gekippt. Die nachgeordneten Schrauben **20** liegen mit einer gewissen Verkippung gegenüber der Horizontalen im Abführabschnitt **19**. Die vorderste Schraube **20** blockiert mit ihrem Schraubenkopf **21** das weitere Nachrücken der nachgeordneten Schrauben **20**. Der Schraubenschaft **22** der vordersten Schraube **20** ist bereits teilweise in eine Schrauben-Aufnahmeausnehmung **33** des Schrauben-Aufnahmeteils **32** eingefügt.

### Figur 18

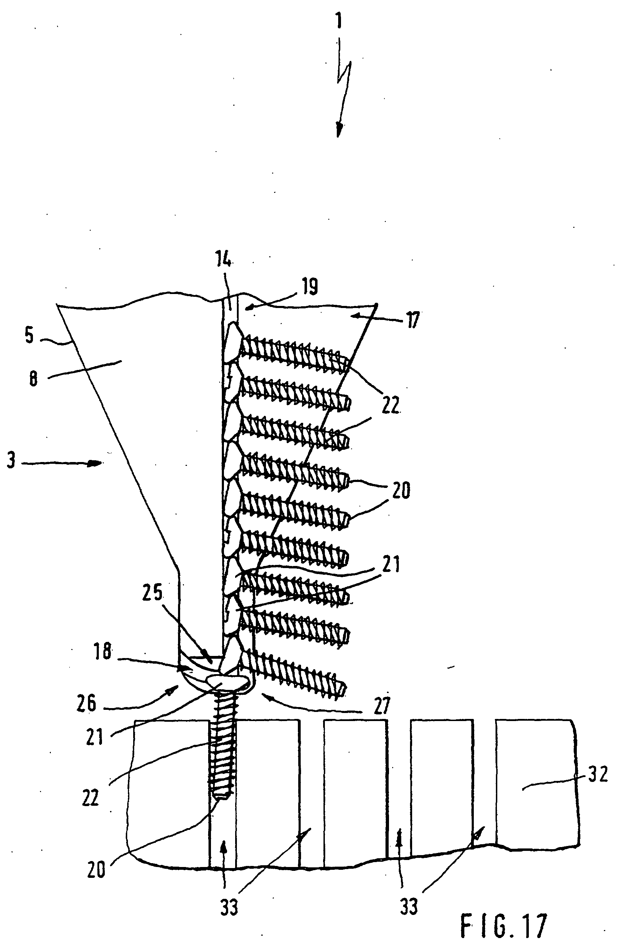

Hier befindet sich das Handstück **1** in einer gegenüber der Vertikalen überkippten Stellung. Die vorderste Schraube **20** steckt im vom Abführabschnitt **19** wegweisenden Austrittsbereich des Endabschnitts **18**, wo die Ausgabesperre durch manuelles abrollartiges Handhaben des Handstücks **1** unter Festlegen des Schraubenschaftes **22** in der Schrauben-Aufnahmeausnehmung **33** bereits überwunden ist. Die Schraube **20** ist mit ihrem Schraubenkopf **21** aufgrund des Überkippens des Handstücks **1** gegenüber der Vertikalen grösstenteils bereits aus dem Endabschnitt **18** der Kopf-Führungsnut **14** ausgetreten. Die nächstfolgende Schraube **20** ist mit ihrem Schraubenkopf **21** noch im Übergangsbereich zwischen dem Abführabschnitt **19** und dem Endabschnitt **18** verklemmt. Nach Abziehen des Handstücks **1** vom Schraubenkopf **21** der in eine Schrauben-Aufnahmeausnehmung **33** eingefügten Schraube **20** und einem erneuten, im wesentlichen vertikalen Ausrichten des Handstückes **1** rutscht die nächste Schraube **20** in die Stellung gemäss Figur 17. Somit ist das Schrauben-Aufnahmeteil **32** mit dieser nächsten Schraube **20** bestückbar.

### Figur 19

Jetzt ist die Kopf-Führungsnut **14** frei von Schrauben **20** und das Handstück **1** gegenüber der Vertikalen überkippt. Der Schraubenkopf **21** einer fälschlicherweise eingefügten Schraube **20** - mit gegenüber benachbarten Schrauben **20** kürzerem Schraubenschaft **22** - ist im dem Abführabschnitt **19** gegenüberliegenden Endbereich des Endabschnitts **18** angeordnet und bereits teilweise in diesen eingetreten.

### Figur 20

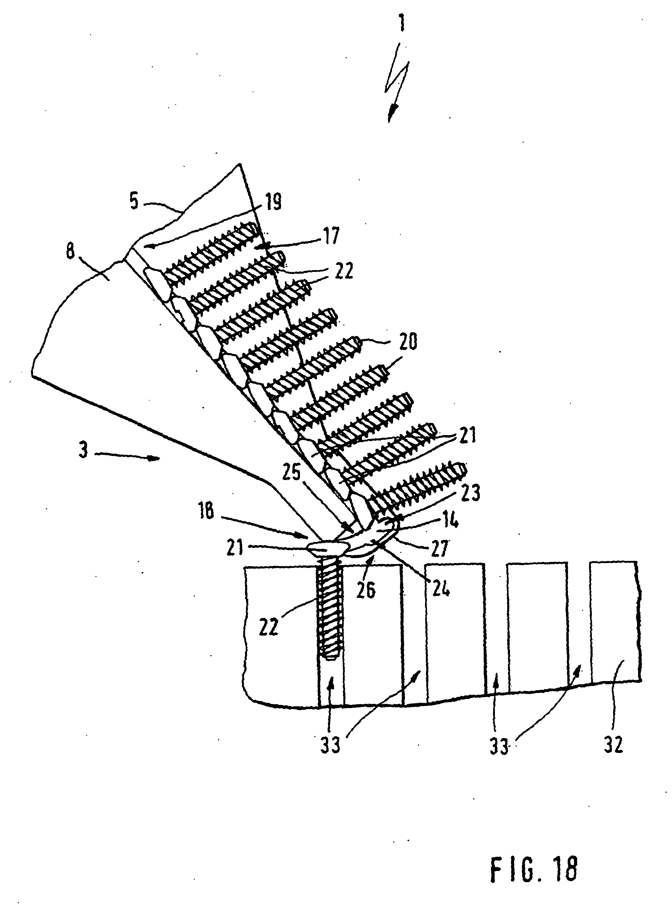

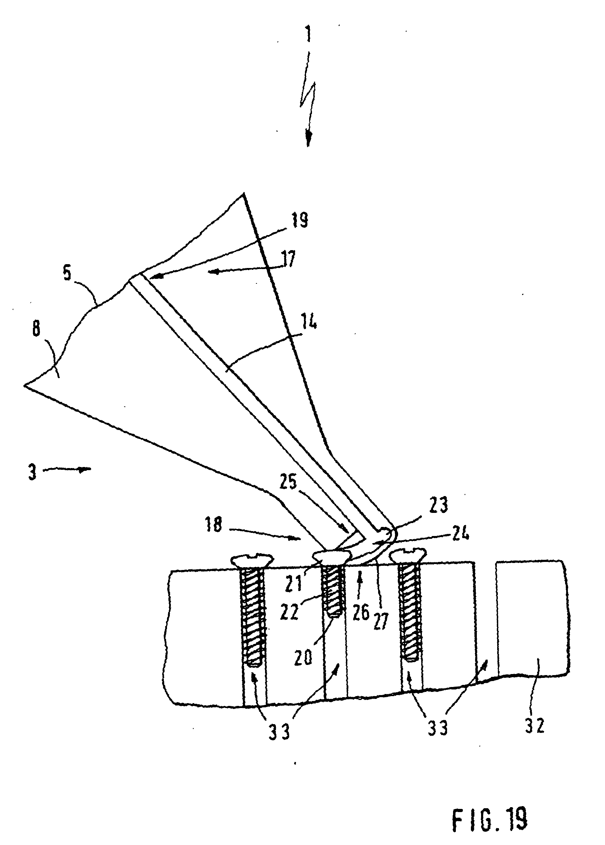

Das Handstück **1** befindet sich in einer im wesentlichen rechtwinklig auf das Schrauben-Aufnahmeteil **32** ausgerichteten vertikalen und vom Schrauben-Aufnahmeteil **32** abgehobenen Stellung. Die Schraube **20** mit kürzerem Schraubenschaft **22** ist mit ihrem Schraubenkopf **21** in die Ausbuchtung **24** sowie den Sackfortsatz **23** im Übergangsbereich zwischen dem Endabschnitt **18** und dem Abführabschnitt **19** der Kopf-Führungsnuten **14** eingetreten. In dieser Anordnung ist die Schraube **20** sicher gehalten und vom Schrauben-Aufnahmeteil **32** entnehmbar sowie beispielsweise in ein anderes Schrauben-Aufnahmeteil **32**, entsprechend der zu den Figuren 17 bis 19 erläuterten Vorgehensweise, korrekt einsetzbar.

## Patentansprüche

1. Vorrichtung zum Handhaben von Gegenständen (**20**) mit einem Kopf (**21**), einem Schaft (**22**) und einer soliden Aussenkontur, wie Schrauben, Nägel und dergleichen, mit
a) einem Handstück (**1**), das eine Kammer (**2**) mit einem Grund (**13**) zur Aufnahme der Gegenstände (**20**) aufweist, wobei
b) sich von einer Innenseite (**11**) der Kammer (**2**) zu einer Ausgabeseite (**3**) des Handstücks (**1**) eine Führungsausnehmung (**14,17**) erstreckt, die einen an die Aussenkontur der Gegenstände (**20**) angepassten Querschnitt aufweist, und am ausgabeseitigen Ende über einen Endabschnitt (18) verfügt, der gegenüber einem an die Kammer (**2**) angrenzenden Abführabschnitt (**19**) abgewinkelt ist, **dadurch gekennzeichnet, dass** der Endabschnitt (**18**)
c) wenigstens im an den Abführabschnitt (**19**) angrenzenden Bereich spitzwinklig zum Abführabschnitt **(19)** ausgerichtet ist; und
d) entgegen der Zuführrichtung eines Gegenstands (**20**) von der Kammer (**2**) in den an den Endabschnitt (**18**) angrenzenden vorderseitigen Bereich des Abführabschnitts (**19**), also in Richtung des Abführabschnitts (**19**), geneigt verläuft.

2. Vorrichtung nach Anspruch 1, **dadurch gekennzeichnet, dass** der Endabschnitt (**18**) in Richtung des Abführabschnitts (**19**) gekrümmt verläuft.

3. Vorrichtung nach Anspruch 1 oder 2, **dadurch gekennzeichnet, dass**
a) im Übergangsbereich zwischen dem Abführabschnitt (**19**) und dem Endabschnitt (**18**) ein sich in Verlängerung des Endabschnitts (**18**) erstreckender Sackfortsatz (**23**) ausgebildet ist; und
b) der Endabschnitt (**18**) im Stossbereich mit dem Abführabschnitt (**19**) eine dem Abführabschnitt (**19**) gegenüberliegende Ausbuchtung (**24**) aufweist.

4. Vorrichtung zum Handhaben von Gegenständen (**20**) mit einem Kopf (**21**), einem Schaft (**22**) und einer soliden Aussenkontur, wie Schrauben, Nägel und dergleichen, mit
a) einem Handstück (**1**), das eine Kammer (**2**) mit einem Grund (**13**) zur Aufnahme der Gegenstände (**20**) aufweist, wobei
b) sich von einer Innenseite (**11**) der Kammer (**2**) zu einer Ausgabeseite (**3**) des Handstücks (**1**) eine Führungsausnehmung (**14**,**17**) erstreckt, die einen an die Aussenkontur der Gegenstände (**20**) angepassten Querschnitt aufweist, und am ausgabeseitigen Ende über einen Endabschnitt (**18**) verfügt, der gegenüber einem an die Kammer (**2**) angrenzenden Abführabschnitt (**19**) abgewinkelt ist, **dadurch gekennzeichnet, dass**
c) der Endabschnitt (**18**) wenigstens in seinem an den Abführabschnitt (**19**) angrenzenden Bereich rechtwinklig zum Abführabschnitt (**19**) ausgerichtet ist; und
d) im Übergangsbereich zwischen dem Abführabschnitt (**19**) und dem Endabschnitt (**18**), als ein Kopf-Einrastmittel, ein sich in Verlängerung des Endabschnitts (**18**) erstreckender Sackfortsatz (**23**) und/oder eine dem Abführabschnitt (**19**) gegenüberliegende Ausbuchtung (**24**) vorgesehen sind.

5. Vorrichtung nach einem der Ansprüche 1 bis 4, **dadurch gekennzeichnet, dass**
a) im Übergangsbereich zwischen dem Abführabschnitt (**19**) und dem Endabschnitt (**18**) eine Übergangsausnehmung (**25**) eingebracht ist; und
b) zwischen dem Abführabschnitt (**19**) und dem Endabschnitt (**18**) ein gekrümmter Übergangsabschnitt (**28**) vorgesehen ist.

6. Vorrichtung nach einem der Ansprüche 1 bis 5, **dadurch gekennzeichnet, dass**
a) die Führungsausnehmung (**14,17**) mit ihrem die Schäfte (**22**) der Gegenstände (**20**) aufnehmenden Teil (**17**) zu der der Kammer (**2**) gegenüberliegenden Aussenseite des Handstücks (1) geöffnet ist; und
b) die Führungsausnehmung (**14,17**) in den Grund (**13**) der Kammer (**2**) mündet und sich über den Grund (**13**) der Kammer (**2**) erstreckt.

7. Vorrichtung nach Anspruch 6, **dadurch gekennzeichnet, dass**
a) die Kammer (**2**) eine Wandung (**9**,**10**) besitzt, welche wenigstens im Bereich des Grunds (**13**) spitzwinklig ausgebildet ist; und
b) die Führungsausnehmung (**14,17**) im Mündungsbereich (**15**) in die Kammer (**2**) mittels einer Abrundung (**16**) im Querschnitt aufgeweitet ist.

8. Vorrichtung nach einem der Ansprüche 1 bis 7, **dadurch gekennzeichnet, dass** das Handstück (**1**) aus zwei aneinander fügbaren Handstückteilen (**5**) aufgebaut ist, wobei die Führungsausnehmung durch in die Handstückteile (**5**) eingebrachte Kopf-Führungsnuten (**14**) zur Aufnahme der Köpfe (**21**) und Schaftführungs-Ausnehmungen (**17**) zur Aufnahme der Schäfte (**22**) der Gegenstände (**20**) gebildet ist.

9. Vorrichtung nach einem der Ansprüche 1 bis 8, **dadurch gekennzeichnet, dass**
a) das Handstück (**1**) länglich ausgebildet und die Ausgabeseite (**3**) spitzenartig verjüngt ist; und
b) die Kammer (**2**) mit einem Deckel (**6**) verschliessbar ist.

## Claims

1. Device for handling items (**20**) with a head (**21**), a shank (**22**) and a solid outer contour, such as screws, pins and the like, having
a) a hand piece (**1**), which has a chamber (**2**) with a base (**13**) for receiving the items (**20**),
b) there extending from the inner side (**11**) of the chamber (**2**) to a dispensing side (**3**) of the hand piece (**1**) a guiding recess (**14**,**17**), which has a cross-section adapted to the outer contour of the items (**20**) and has at the end on the dispensing side an end portion (**18**) which is angled away with respect to a delivery portion (**19**) adjoining the chamber (**2**), **characterized in that** the end portion (**18**)
c) at least in the region adjoining the delivery portion (**19**), is aligned at an acute angle with respect to the delivery portion (**19**); and
d) runs at an inclination counter to the feeding direction of an item (**20**) from the chamber (**2**) into the front region of the delivery portion (**19**) adjoining the end portion (**18**), thus in the direction of the delivery portion (**19**).

2. Device according to Claim 1, **characterized in that** the end portion (**18**) is curved in the direction of the delivery portion (**19**).

3. Device according to Claim 1 or 2, **characterized in that**
a) in the transitional region between the delivery portion (**19**) and the end portion (**18**) there is formed a blind continuation (**23**) extending in prolongation of the end portion (**18**); and
b) the end portion (**18**) has in the region where it meets the delivery portion (**19**) a depression (**24**) lying opposite the delivery portion (**19**).

4. Device for handling items (**20**) with a head (**21**), a shank (**22**) and a solid outer contour, such as screws, pins and the like, having
a) a hand piece (**1**), which has a chamber (**2**) with a base (**13**) for receiving the items (**20**),
b) there extending from an inner side (**11**) of the chamber (**2**) to a dispensing side (**3**) of the hand piece (**1**) a guiding recess (**14**,**17**), which has a cross-section adapted to the outer contour of the items (20) and has at the end on the dispensing side an end portion (**18**) which is angled away with respect to a delivery portion (**19**) adjoining the chamber (**2**), **characterized in that**
c) the end portion (**18**), at least in its region adjoining the delivery portion (**19**), is aligned at right angles with respect to the delivery portion (**19**); and
d) in the transitional region between the delivery portion (**19**) and the end portion (**18**), a blind continuation (**23**) extending in prolongation of the end portion (**18**) and/or a depression (**24**) lying opposite the delivery portion (**19**) is provided as a head-engaging means.

5. Device according to one of Claims 1 to 4, **characterized in that**
a) in the transitional region between the delivery portion (**19**) and the end portion (**18**) there is provided a transitional recess (**25**); and
b) between the delivery portion (**19**) and the end portion (**18**) there is provided a curved transitional portion (**28**).

6. Device according to one of Claims 1 to 5, **characterized in that**
a) with its part (**17**) receiving the shanks (**22**) of the items (**20**), the guiding recess (**14**,**17**) is open with respect to the outer side of the hand piece (**1**) lying opposite the chamber (**2**); and
b) the guiding recess (**14**,**17**) enters the base (**13**) of the chamber (**2**) and extends over the base (**13**) of the chamber (**2**).

7. Device according to Claim 6, **characterized in that**
a) the chamber (**2**) has a wall (**9,10**) which, at least in the region of the base (**13**), is formed at an acute angle; and
b) in the entry region (**15**) into the chamber (**2**), the guiding recess (**14,17**) is widened in cross-section by means of a rounding-off (**16**).

8. Device according to one of Claims 1 to 7, **characterized in that** the hand piece (**1**) is made up of two hand piece parts (**5**) which can be fitted together, the guiding recess being formed by head-guiding grooves (**14**), for receiving the heads (**21**), and shank-guiding recesses (**17**), for receiving the shanks (**22**) of the items (**20**), provided in the hand piece parts (**5**).

9. Device according to one of Claims 1 to 8, **characterized in that**
a) the hand piece (**1**) is of an elongated design and the dispensing side (**3**) is tapered in a tip-like manner; and
b) the chamber (**2**) can be closed by a cover (**6**)

## Revendications

1. Dispositif de manipulation d'articles (20) avec une tête (21), une tige (22) et un contour extérieur solide, comme des vis, des clous ou similaires, avec
a) un outil à main (1), qui présente une chambre (2) avec un fond (13) destinée à recevoir les articles (20), dans lequel
b) une cavité de guidage (14, 17) s'étend d'une face intérieure (11) de la chambre (2) jusqu'à une face de sortie (3) de l'outil à main (1), cavité qui présente une section transversale adaptée au contour extérieur des articles (20) et qui dispose, à l'extrémité de sortie, d'une partie terminale (18) qui est coudée par rapport à une partie d'évacuation (19) jointive à la chambre (2), **caractérisé en ce que** la partie terminale (18)
c) est orientée sous un angle aigu vers la partie d'évacuation (19) au moins dans la région jointive à la partie d'évacuation (19); et
d) est inclinée dans le sens contraire à la direction d'arrivée d'un article (20) depuis la chambre (2) jusque dans la région avant de la partie d'évacuation (19), jointive à la partie terminale (18), donc en direction de la partie d'évacuation (19).

2. Dispositif selon la revendication 1, **caractérisé en ce que** la partie terminale (18) est courbée en direction de la partie d'évacuation (19).

3. Dispositif selon la revendication 1 ou 2, **caractérisé en ce que**
a) un prolongement borgne (23) s'étendant dans le prolongement de la partie terminale (18) est formé dans la région de transition entre la partie d'évacuation (19) et la partie terminale (18); et
b) la partie terminale (18) présente, dans la région de jonction avec la partie d'évacuation (19), un renflement (24) situé en face de la partie d'évacuation (19).

4. Dispositif de manipulation d'articles (20) avec une tête (21), une tige (22) et un contour extérieur solide, comme des vis, des clous ou similaires, avec
a) un outil à main (1), qui présente une chambre (2) avec un fond (13) destinée à recevoir les articles (20), dans lequel
b) une cavité de guidage (14, 17) s'étend d'une face intérieure (11) de la chambre (2) jusqu'à une face de sortie (3) de l'outil à main (1), cavité qui présente une section transversale adaptée au contour extérieur des articles (20) et qui dispose, à l'extrémité de sortie, dune partie terminale (18) qui est coudée par rapport à une partie d'évacuation (19) jointive à la chambre (2), **caractérisé en ce que**
c) la partie terminale (18) est orientée sous un angle droit vers la partie d'évacuation (19) au moins dans sa région jointive à la partie d'évacuation (19); et
d) il est prévu, dans la région de transition entre la partie d'évacuation (19) et la partie terminale (18), comme moyen d'accrochage des têtes, un prolongement borgne (23) s'étendant dans le prolongement de la partie terminale (18) et/ou un renflement (24) situé en face de la partie d'évacuation (19).

5. Dispositif selon l'une quelconque des revendications 1 à 4, **caractérisé en ce que**
a) une cavité de transition (25) est ménagée dans la région de transition entre la partie d'évacuation (19) et la partie terminale (18); et
b) il est prévu une partie de transition courbée (28) entre la partie d'évacuation (19) et la partie terminale (18).

6. Dispositif selon l'une quelconque des revendications 1 à 5, **caractérisé en ce que**
a) la cavité de guidage (14, 17) avec sa partie (17) recevant les tiges (22) des articles (20) est ouverte vers la face extérieure de l'outil à main (1) située en face de la chambre (2); et
b) la cavité de guidage (14, 17) débouche dans le fond (13) de la chambre (2) et s'étend sur le fond (13) de la chambre (2).

7. Dispositif selon la revendication 6, **caractérisé en ce que**
a) la chambre (2) possède une paroi (9, 10), qui est façonnée à angle aigu au moins dans la région du fond (13); et
b) la cavité de guidage (14, 17) a une section transversale agrandie par un arrondi (16) dans la région de l'embouchure (15) dans la chambre (2).

8. Dispositif selon l'une quelconque des revendications 1 à 7, **caractérisé en ce que** l'outil à main (**1**) est constitué de deux parties d'outil (5) à assembler l'une à l'autre, dans lequel la cavité de guidage est formée par des rainures de guidage des têtes (14) destinées à recevoir les têtes (21) et par des cavités de guidage de tige (17) destinées à recevoir les tiges (22) des articles (20), ménagées dans les parties (5) de l'outil à main.

9. Dispositif selon l'une quelconque des revendications 1 à 8, **caractérisé en ce que**
a) l'outil à main (1) est de forme allongée et la face de sortie (3) se rétrécit en pointe; et
b) la chambre (2) peut être fermée par un couvercle (6).
